# EUROPEAN PATENT APPLICATION

(11) **EP 4 438 741 A1**
(43) Date of publication of application: **02.10.2024**
(21) Application number: 22714760.0
(22) Date of filing: 21.02.2022
(51) Int. Cl.: C12Q 1/6869

(54) **HIGH-THROUGHPUT SEQUENCING METHOD BASED ON INTERNAL REFERENCE OF KNOWN TAG**

(30) Priority: 26.11.2021 CN 202111422884
(71) Applicant: Daan Gene Co., Ltd., Guangzhou, Guangdong 510665 (CN)
(72) Inventor: The designation of the inventor has not yet been filed
(74) Representative: Handsome I.P. Ltd
(86) International application number: PCT/CN2022/077072
(87) International publication number: WO 2023/092872

(57) **Abstract**

Embodiments of the present disclosure, pertaining to the technical field of high-throughput sequencing, relate to a method for high-throughput sequencing based on an internal reference with a known index. The method includes: generating a random DNA sequence, adding a single-ended adapter DNA sequence containing the known index at both ends of the random DNA sequence to obtain an internal reference sequence, and synthesizing a sequencing quality control sequence based on the internal reference sequence; performing, based on the sequencing quality control sequence, high-throughput sequencing on a library of samples to be tested to obtain sequencing data; and performing result analysis on the sequencing data to obtain a sample error distribution rate of the library of samples to be tested, and ending the high-throughput sequencing. According to the present disclosure, index hopping in the process of sequencing may be monitored based on the internal reference.

## Description

This application is based upon and claims priority to Chinese Patent Application No. 202111422884.6, filed before China National Intellectual Property Administration on November 26, 2021 and entitled "METHOD FOR HIGH-THROUGHPUT SEQUENCING BASED ON INTERNAL REFERENCE WITH KNOWN INDEX," the content of which is incorporated herein by reference in its entirety.

### TECHNICAL FIELD

The present disclosure relates to the technical field of high-throughput sequencing, and in particular, relates to a method for high-throughput sequencing based on an internal reference with a known index.

### BACKGROUND

With the development of genomics technology, high-throughput sequencing technology, also referred to as next-generation sequencing (NGS), has been widely used in infectious disease prevention and control, such as the investigation of outbreaks of infectious diseases in hospitals, identification of unknown pathogens, detection of resistance gene mutations in pathogens; early diagnosis and precise treatment of tumors (for example, lung cancer, breast cancer, gastrointestinal tumors, melanoma, and the like), such as detection of driver gene mutations associated with individualized tumor treatment, tumor genomics research, and exploration on tumor heterogeneity, drug resistance and tumor clonal evolution process and mechanism; early screening and diagnosis of genetic diseases, such as genetic disease diagnosis, neonatal screening, prenatal screening, pre-implantation screening and other fields, such as genetic deafness and non-invasive screening. The high-throughput sequencing technology is under rapid developments in the direction of convenience and economy. By high-throughput sequencing, i.e., the sequencing of tens of thousands of DNA molecules at the same time, more samples are mixed and loaded, samples are typically distinguished from each other in library preparation by means of adapters (Y-adapters, U-adapters, blunt-ended adapters, and bubble-adapters) or PCR amplification introduced tags (barcodes or indexes).

Studies have found that sequencing platforms based on ExAmp (exclusive amplification), such as HiSeq 3000/4000, HiSeq X Ten and NovaSeq, have the problems of index error distribution (i.e., index hopping) in mixing and loading sequencing of the samples, with a sample error distribution rate exceeding 1% and index hopping rates as high as 6% for PCR-free libraries. Even with the cumbersome nonconbinatorial dual index solution, the index contamination rate may only be reduced by 0.08%.

In June 2018, relevant researchers from Shenzhen BGI studied the problem of index hopping of the DNB sequencing platform by three mainstream library preparation methods. The BGISEQ sequencer utilizes the unique DNA nanoball (DNB) sequencing technology to perform library amplification based on rolling circle replication (RCR). This linear amplification may avoid error accumulation associated with the conventional PCR. The DNB-based NGS application achieves a low sample error distribution rate as low as 0.0001% to 0.0004% using only a single index. In addition, water is used instead of DNA, a index is added, a blank control is added, and the probability of mismatch on the DNB sequencing platform is one in 36 million reads, i.e., 0.0000028%. For the PCR-free library, an average contamination rate is about 0.0004%.

The inventors have found that while the index mismatch rate is lower on the BGI platform compared to the Illumina platform, index hopping is present on both platforms. More importantly, it is difficult to monitor index hopping.

### SUMMARY

An object of embodiments of the present disclosure is to provide a method for high-throughput sequencing based on an internal reference with a known index, which is capable of monitoring index hopping in the process of sequencing based on the internal reference.

In view of the above, the embodiments of the present disclosure provide a method for high-throughput sequencing based on an internal reference with a known index. The technical solutions are as follows:
A method for high-throughput sequencing based on an internal reference with a known index includes:
generating a random DNA sequence, adding a single-ended adapter DNA sequence containing the known index at both ends of the DNA sequence to obtain an internal reference sequence, and synthesizing a sequencing quality control sequence based on the internal reference sequence;
performing, based on the sequencing quality control sequence, high-throughput sequencing on a library of samples to be tested to obtain sequencing data;
performing result analysis on the sequencing data to obtain a sample error distribution rate of the library of samples to be tested, and ending the high-throughput sequencing.

Compared with the related art, the embodiments of the present disclosure mainly achieve the following beneficial effects:
According to the present disclosure, based on the internal reference sequence containing the known index, index hopping may be effectively monitored, index hopping is reflected by data, and the experimenter is helped to analyze causes of index hopping, such that the experimental scheme is adjusted.

### BRIEF DESCRIPTION OF THE DRAWINGS

For clearer descriptions of technical solutions according to the embodiments of the present disclosure, drawings that are to be referred for description of the embodiments are briefly described hereinafter. Apparently, the drawings described hereinafter merely illustrate some embodiments of the present disclosure. Persons of ordinary skill in the art may also derive other drawings based on the drawings described herein without any creative effort.
FIG. 1 is a flowchart of a method for high-throughput sequencing based on an internal reference with a known index according to the present disclosure;
FIG. 2 is a schematic diagram illustrating a result of first-generation sequencing on a sequencing quality control sequence according to the present disclosure;
FIG. 3 is a schematic diagram illustrating another result of first-generation sequencing on the sequencing quality control sequence according to the present disclosure;
FIG. 4 is a schematic diagram illustrating another result of first-generation sequencing on the sequencing quality control sequence according to the present disclosure;
FIG. 5 is a schematic diagram illustrating another result of first-generation sequencing on the sequencing quality control sequence according to the present disclosure;
FIG. 6 is a schematic diagram illustrating a result of electrophoresis on an amplified sequencing quality control sequence according to the present disclosure;
FIG. 7a is a schematic diagram illustrating a result of Qsep analysis on the amplified sequencing quality control sequence according to the present disclosure;
FIG. 7b is a schematic diagram illustrating another result of Qsep analysis on the amplified sequencing quality control sequence according to the present disclosure;
FIG. 7c is a schematic diagram illustrating another result of Qsep analysis on the amplified sequencing quality control sequence according to the present disclosure; and
FIG. 7d is a schematic diagram illustrating another result of Qsep analysis on the amplified sequencing quality control sequence according to the present disclosure.

### DETAILED DESCRIPTION

Unless defined otherwise, all technical and scientific terms used herein have the same meaning as commonly understood by one of ordinary skill in the art to which this application pertains. The terms used herein in the specification of present disclosure are only intended to illustrate the specific embodiments of the present disclosure, instead of limiting the present disclosure. The terms "comprise," "include," and any variations thereof in the specification and claims of the present disclosure and in the description of the drawings are intended to cover a non-exclusive inclusion. Terms such as "first," "second," and the like in the specification, claims, or the accompanying drawings of the present disclosure are intended to distinguish different objects, but are not intended to define a specific sequence.

The term "embodiment" in this specification signifies that the specific characteristic, structures or features described with reference to the embodiments may be covered in at least one embodiment of the present disclosure. This term, when appears in various positions of the specification, neither indicates the same embodiment, nor indicates an independent or optional embodiment that is exclusive of the other embodiments. A person skilled in the art would implicitly or explicitly understand that the embodiments described in this specification may be incorporated with other embodiments.

The following embodiments are given for better understanding of the present disclosure, rather than for limiting the present disclosure. In the embodiments described hereinafter, the experimental methods, unless otherwise specified, are all routine methods. In the embodiments described hereinafter, the experimental materials, unless otherwise specified, are all purchased from common biochemical reagent suppliers.

To make a person skilled in the art better understand the technical solutions of the embodiments of the present disclosure, the technical solutions of the present disclosure are clearly and completely described with reference to the accompanying drawings of the embodiments of the present disclosure.

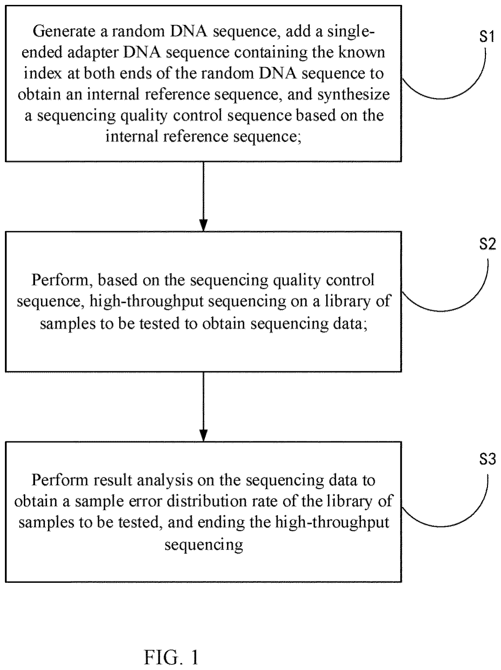

Still referring to FIG. 1, a flowchart of a method for high-throughput sequencing based on an internal reference with a known index according to one embodiment of the present disclosure is illustrated. The method for high-throughput sequencing based on the internal reference with the known index includes the following steps:
S1: generating a random DNA sequence, adding a single-ended adapter DNA sequence containing the known index at both ends of the random DNA sequence to obtain an internal reference sequence, and synthesizing a sequencing quality control sequence based on the internal reference sequence;
S2: performing, based on the sequencing quality control sequence, high-throughput sequencing on a library of samples to be tested to obtain sequencing data; and
S3: performing result analysis on the sequencing data to obtain a sample error distribution rate of the library of samples to be tested, and ending the high-throughput sequencing.

In this embodiment, the single-ended adapter sequence is a single-ended adapter sequence with a known index provided by the BGI Group. The sequencing platform used in high-throughput sequencing on the library of samples to be tested by the sequencing quality control sequence is a sequencer manufactured by the BGI Group, and MGI 2000 sequencing is performed. According to the present disclosure, index hopping may be monitored based on an internal reference sequence with a known index, and index hopping is reflected by data. In this way, the experimenter is helped to analyze the causes and adjust the experimental scheme.

It should be noted that the specific single-ended adapter sequence according to the present disclosure includes, but is not limited to, the single-ended adapter sequence provided by the BGI Group, and the sequencing platform used includes, but is not limited to, the sequencer manufactured by the BGI Group. Alternatively, an adapter sequence supplied by Illumina and a corresponding sequencer manufactured by Illumina may also be used.

### I. Generation and screening of random DNA sequence:

Specifically, generating the random DNA sequence includes:
selecting a reverse virus sequence by a specific species screening algorithm; and
cutting the selected reverse virus sequence into a preset size, making a comparison against a pathogen database and a host database, and determining the reverse virus sequence as the random DNA sequence in the case that the selected reverse virus sequence is not present in the pathogen database or the host database.

In this embodiment, a screening process of a random sequence is as follows: a reverse virus sequence is directly selected by a species-specific screening algorithm, then the selected sequence is cut into 150 bp by jellyfish, the pathogen database and the host database were aligned by BLASTN, and in the case that the alignment is not up to a host and any species, the sequence is determined as the random sequence.

A fragment size of the random sequence generated in the present disclosure is a size of the fragment in the library of samples to be tested to be tested minus a size of the two single-ended adapter sequences. That is, it can be seen that the fragment size of the internal reference sequence generated according to the random sequence is determined according to the size of the fragment in the library of samples to be tested. For example, the fragment size in the library of samples to be tested in the present disclosure is about 250 bp, at this moment, the fragment size of the random sequence is set as 150 bp, a specific single-ended adapter sequence is added at both ends of the random sequence, and the fragment size of the obtained internal reference sequence is 246 bp. The random sequence is a gene sequence of a non-pathogenic pathogen. The random sequence is a bacteriophage sequence or a phytogenic pathogen sequence, wherein both the bacteriophage sequence and the phytogenic pathogen sequence are gene sequences of a non-pathogenic pathogen.

### II. Synthesis of sequencing quality control sequence: a BGI single-ended adapter is added and cloned into a pUC57 vector.

Synthesizing the sequencing quality control sequence based on the internal reference sequence includes:
cloning the internal reference sequence into the pUC57 vector, and synthesizing the sequencing quality control sequence.

### 1. Synthesis of sequencing quality control sequence (i.e., internal reference sequence plasmid):

Sequences CS0002, CS0003, CS0004, and CS0005 in Table 1 are synthesized by Sangon Biotech (Shanghai) Co. Ltd.

**Table 1 Aggregates of sequencing quality control sequences**

| Serial number | Sequencing quality control sequence |
|---|---|
| CS0002 | gaacgacatggctacgatccgacttcctgggtgagcatcaggggcgatacgttcacatcccggaggacctcgaagcacaacgccaggactgcgaagat ccacgaaatcacgaaacagcacgacattgaaggttggactgatccagttgctgggtatgtatggatataagttcatcaagtcggaggccaagcggtcttaggaag acaataggtccgatcaactccttggctcaca |
| CS0003 | gaacgacatggctacgatccgacttagaatgaagtgggcacagttaagatctaataaaacgtggaaacagtcttgacgggctacgatcagagcgggacat cagtaactaatagccagtctcgtgcgaccgtaaccaagacgctcaccgtaataaccaggaggagtaacggcggggaagtcggaggccaagcggtcttaggaa gacaaggacggaatccaactccttggctcaca |
| CS0004 | gaacgacatggctacgatccgacttttggtgttgttcattttacgaactgcacttgtacatttattacggcgaacgaattatcagtcccgaactgtaatactgact gtaagttagttactaggagtaaattgcgtgagtataatggtaactacgtcatgctggactgtgttaccgaagtcggaggccaagcggtcttaggaagacaacttact gccgcaactccttggctcaca |
| CS0005 | gaacgacatggctacgatccgacttgaggaaccagttcctcatacatacgtttacttaatgacagactcccttatacgtgttaacgttaatctcttagtgttagtg agagtaaagttatatggtcattagataatagagggatgataaaatgtgaatcatcgagagacgatttctaagtcggaggccaagcggtcttaggaagacaaaccta attgacaactccttggctcaca |

In CS0002, gaacgacatggctacgatccgactt and aagtcggaggccaagcggtcttaggaagacaataggtccgatcaactccttggctcaca denote single-ended adapter sequences, and taggtccgat denotes a sequence (10 bp) with a known index ( barcode).

In CS0003, gaacgacatggctacgatccgact and aagtcggaggccaagcggtcttaggaagacaaggacggaatccaactccttggctcaca denotes single-ended adapter sequences, and GGACGGAATC denotes a sequence with a known index.

In CS0004, gaacgacatggctacgatccgactt and aagtcggaggccaagcggtcttaggaagacaacttactgccgcaactccttggctcaca denote single-ended adapter sequences, and CTTACTGCCG denotes a sequence with a known index.

In CS0005, gaacgacatggctacgatccgactt and aagtcggaggccaagcggtcttaggaagacaaacctaattgacaactccttggctcaca denote single-ended adapter sequences, and ACCTAATTGA denotes a sequence with a known index.

### 2. Identification of first-generation sequencing on sequencing quality control sequence (i.e., internal reference sequence plasmid):

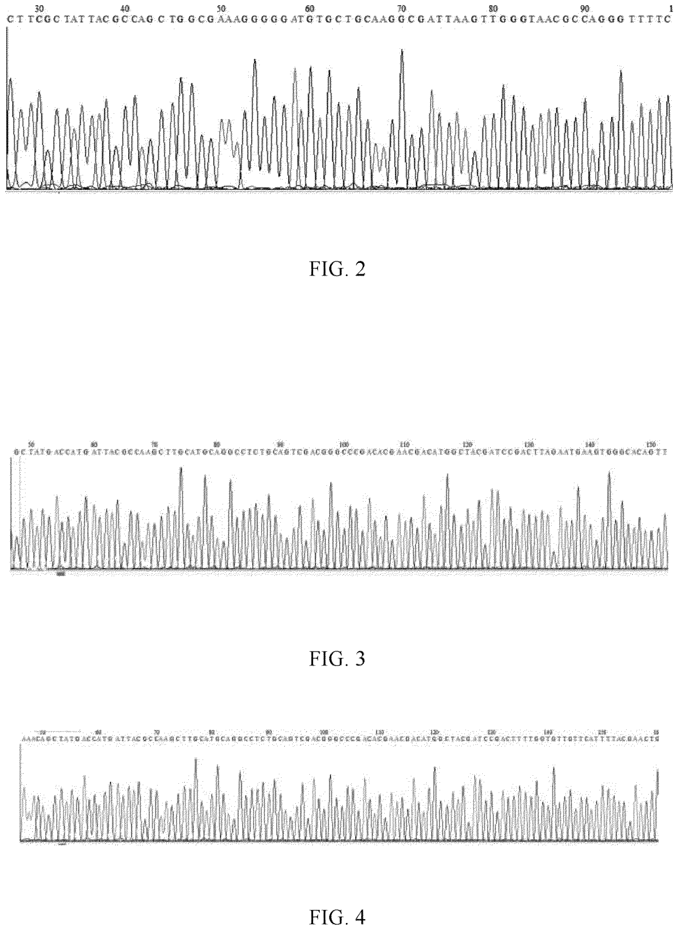

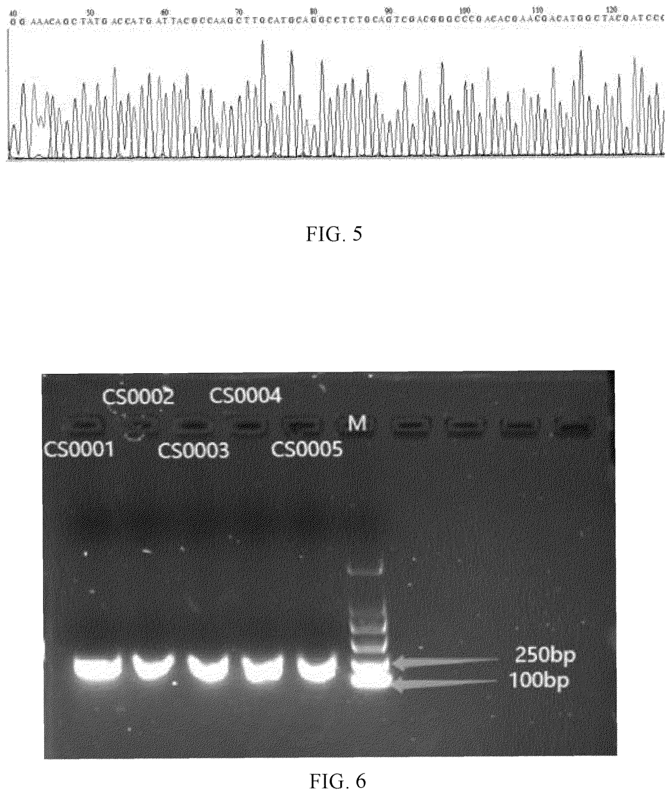

Sequencing results are as illustrated in FIG. 2, FIG. 3, FIG. 4, and FIG. 5. FIG. 2 is a schematic diagram illustrating a result of first-generation sequencing on a sequencing quality control sequence (CS0002). FIG. 3 is a schematic diagram illustrating a result of first-generation sequencing on a sequencing quality control sequence (CS0003). FIG. 4 is a schematic diagram illustrating a result of first-generation sequencing on a sequencing quality control sequence (CS0004). FIG. 5 is a schematic diagram illustrating a result of first-generation sequencing on a sequencing quality control sequence (CS0005).

As seen from the sequencing peak maps in FIG. 2 to FIG. 5, the results of the first-generation sequencing indicate good quality, which indicates that the plasmids are successfully constructed and the subsequent experiments can be continued.

### 3. Amplification of sequencing quality control sequence (i.e., internal reference sequence plasmid):

The sequencing quality control sequence is amplified by a designated PCR amplification primer to obtain a target sequencing quality control sequence.
1) The plasmid is dissolved, 60 ng of the dissolved plasmid is subjected to amplification in accordance with the following reaction system and reaction procedure, and a sequence containing a BGI single barcode.

**Table 2 Standard library amplification reaction system and reaction procedure**

| | | Component | Volume (µL) | |
|---|---|---|---|---|
| First round of library amplification | Reaction system | Plasmid (60 ng) | 2 | |
| | | 5*Q5 buffer | 10 | |
| | | dNTP (10 mM) | 1 | |
| | | Q5^{®} Hot start ultra-fidelity DNA polymerase | 0.5 | |
| | | PCR primer mixture (15 µM each) MGIAd_PCR 1/MGIAd_PCR 2 | 5 | |
| | | ddH2O | 31.5 | |
| | | Total | 50 | |

| | | Temperature | Time | |
|---|---|---|---|---|
| | Reaction procedure | Hot cover | 105°C | |
| | | 95°C | 3 min | 1 |
| | | 95°C | 20s | 30 |
| | | 58°C | 20s | |
| | | 72°C | 20s | |
| | | 72°C | 5min | 1 |
| | | 4°C | Hold | |
| Gel extraction | Operate according to the instructions of Invitrogen Purelink Rapid Gel Extraction Kit | | | |

2) Agarose electrophoresis

Whether an amplification product is consistent with an expected product is detected by agarose gel electrophoresis. Specifically, an agarose gel is formulated, a sample is added into the agarose gel, and an electrophoresis experiment is carried out by an electrophoresis apparatus to obtain an electrophoresis result image; and whether a band position is consistent with an expected position is determined according to the electrophoresis result image.

1.2 g of agarose is added into 100 mL of 1X TAE electrophoresis buffer, and shaken well. The solution is heated in a microwave oven until the agarose is completely dissolved. The solution is cooled to 60°C, 6 µL Gelred fluorescent dye is added, and the solution is shaken well.

The dissolved agarose is added into a combed gel plate and solidified by cooling at room temperatures.

The gel is placed in an electrophoresis tank, 1X TAE electrophoresis buffer is added until the gel is covered by the buffer by a height of 1-2 mm, and the comb is carefully pulled upwards vertically.

2 µL of PCR amplification product is pipetted onto a parafilm, 2 µL of 3 x Loading Buffer is added and mixed well, and carefully added into spotting wells, and 6 µL DNA marker (DL2000 of Tiangen) is added into the last well.

The power switch is turned on, the voltage is adjusted to 150 V, and the electrophoresis time is 30 min. Through observation, the band of bromophenol blue moves from a negative electrode to a positive electrode.

Upon completion of electrophoresis, imaging is carried out by a Biorad gel imager, a picture obtained by imaging is saved, and a picture of the electrophoresis result upon amplification (upon purification) is shown in FIG. 6. FIG. 6 is a schematic diagram illustrating a result of electrophoresis according to the present disclosure. As seen from FIG. 6, upon purification of the amplified product, the band is single, with a band position around 250 bp, which is consistent with an expected position.

### 3) Qsep analysis

A concentration of the products of PCR amplification obtained in 2) is measured by Qubit, and the concentrations of CS0002, CS0003, CS0004 and CS0005 are respectively: 12.5, 14.7, 21.6, and 19.2 ng/µL.

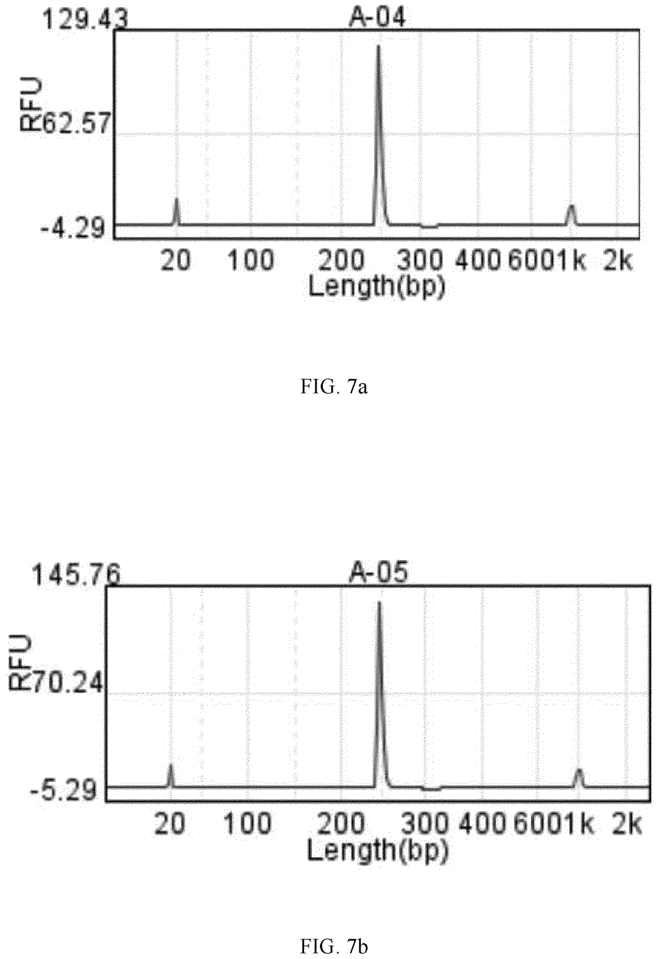

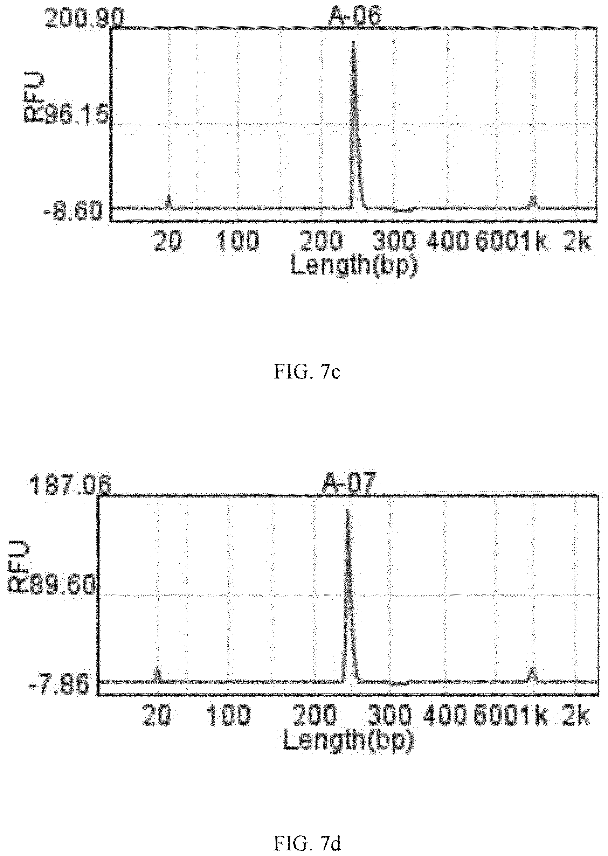

The sample is diluted to a loading concentration (1-2 ng/µL) recommended by Qsep 100, and then fragment size analysis is performed according to the instructions for Qsep 100. The obtained results are shown in FIGS. 7a-7d, which are schematic diagrams showing the results of Qsep analysis on PCR amplification products according to the present disclosure, and FIGS. 7a-7d sequentially illustrate: CS0002, CS003, CS0004, and CS0005. It can be seen from FIGS. 7a-7d that the fragment sizes of fragments CS0002, CS003, CS0004, and CS0005 are between 200 and 300 bp, which are consistent with expected sizes. This indicates that the sequencing quality control sequence is successfully amplified and can be used for subsequent loading testing.

### 4. MGI 200 sequencing of target sequencing quality control sequence together with normal library

An amount of adding the target sequencing quality control sequences is determined to obtain an addition amount, and the target sequencing quality control sequences are added into the library of samples to be tested according to the addition amount to obtain a mixed library; the mixed library is subjected to high-throughput sequencing to obtain the sequencing data.
1) Library pooling: According to the addition amount, the amplified and purified target sequencing quality control sequence is mixed with four standard libraries in an equal amount to form a mixed library, and a concentration of the mixed library is quantitatively determined again.
2) Cyclization:
   A test is carried out according to the instructions for the BGI cyclization kit (Article No.: 1000005259, Kit Version No.: V2.0).
3) DNB preparation and loading sequencing

The DNB is prepared and loading sequencing is carried out according to the instructions for BGI MGISEQ-200 FCL SE 50.

### 5. Data analysis of unloading

Obtaining a sample error distribution rate by performing result analysis on the sequencing data includes:
performing statistical collection based on the sequencing data to obtain the total number of sequenced sequences, the number of internal reference sequences with known indexs, and the number of sequences specifically detected by pathogens; and determining the sample error distribution rate based on the total number of sequenced sequences, the number of internal reference sequences with known indexs, and the number of sequences specifically detected by pathogens. The library of samples to be tested is a pathogen library.

After an unloading sequencing result (fq.gz file) is obtained, data filtering is performed. Specifically, low quality data, adapters, human hosts, and the like are removed. The sequencing data is aligned with the original known sequences by the BWA alignment tool, and analysis is carried out by the pathogen analysis process developed in laboratory. The results are shown in Table 3:

**Table 3-1 Loading sequencing result on sequencing quality control sequence**

| Type of library of samples to be tested | DNA | DNA | DNA | DNA |
|---|---|---|---|---|
| Hepatitis B virus | 9 | 0 | 5 | 4 |
| Escherichia coli | 8 | 0 | 0 | 0 |
| Pseudomonas aeruginosa | 4 | 20 | 8 | 80 |
| Aspergillus fumigatus | 2 | 0 | 0 | 0 |
| Vibrio vulnificus | 2 | 0 | 0 | 0 |
| Hepatitis B virus | 13 | 0 | 0 | 0 |
| Acinetobacter baumannii | 12 | 0 | 5 | 0 |
| Moraxella osloensis | 1 | 0 | 0 | 0 |
| Haemophilus influenzae | 0 | 0 | 0 | 0 |
| Cryptococcus gattii | 0 | 0 | 0 | 0 |
| Streptococcus pneumoniae | 0 | 0 | 0 | 0 |
| Staphylococcus aureus | 0 | 0 | 0 | 0 |
| Epstein-Barr virus | 0 | 0 | 0 | 0 |
| Neisseria flava | 0 | 0 | 2 | 0 |
| Hepatitis B virus | 0 | 10 | 0 | 0 |
| Brucella intermedia | 0 | 5 | 0 | 0 |
| Klebsiella pneumoniae | 0 | 0 | 0 | 0 |
| Porphyromonas gingivalis | 0 | 0 | 0 | 0 |
| Neisseria flavescens | 0 | 0 | 0 | 0 |
| Mycoplasma hyorhinis | 0 | 0 | 0 | 0 |

**Table 3-2 Loading sequencing result on sequencing quality control sequence**

| | CS0002 | CS0003 | CS0004 | CS0005 |
|---|---|---|---|---|
| Host rate | 1.04% | 0.26% | 0.12% | 0.05% |
| Classification rate | 0.01% | 0.01% | 0.00% | 0.05% |
| Known sequence contrast ratio | 52.69% | 49.07% | 62.18% | 60.82% |
| Raw reads (original sequencing data) (M) | 3.31 | 1.99 | 3.57 | 2.43 |
| Coefficient of homogenization to 10 M | 3.02 | 5.03 | 2.8 | 4.12 |
| Concentration (ng/µL) | 14.1 | 11.4 | 16.8 | 16.4 |

As seen from the results in Table 3-1 and Table 3-2, the result of pathogen analysis of each sequencing quality control contains, more or less, the reads (read length, a sequencing sequence obtained from one reaction in high-throughput sequencing) of some pathogens. This indicates that index hopping is present in this batch of data, and the pathogen involved each hopping is related to the sample involved in each pooling. This also indicates that sequencing quality control can be used as an indicator to monitor index hopping. If the hopping species are related to a clinical causative agent, reference may be provided for the interpreter to make the clinical results more accurate.

It should be understood that, although the various steps in the flowcharts of drawings are shown in order as indicated by the arrows, the steps are not necessarily performed in the order indicated by the arrows. The steps are performed in no strict order unless explicitly stated herein, and may be performed in other orders. Furthermore, at least some of the steps in the flowcharts of the drawings may include sub-steps or stages, which are not necessarily performed at the same time, but may be performed at different times, or in different orders, and may be performed in turn or in alternation with at least some of the other steps or sub-steps or stages of other steps.

It is apparent that the embodiments described above are only exemplary ones, but not all embodiments of the present disclosure, and that the attached drawings illustrate exemplary embodiments of the present disclosure but do not limit the scope of the present disclosure. The present disclosure may be embodied in many different forms and, on the contrary, these embodiments are provided to make the disclosure of the present disclosure more thoroughly and completely understood. Although the present disclosure has been described in detail with reference to the above-mentioned embodiments, those skilled in the art will be able to make modifications to the technical solutions disclosed in the above-mentioned specific embodiments or make equivalent substitutions for some of the technical features. Any equivalent structure made based on the specification and accompanying drawings of the present disclosure, even if being directly or indirectly applied to some other related technical fields, shall all fall within the protection scope of the present disclosure.

## Claims

1. A method for high-throughput sequencing based on an internal reference with a known index, comprising:
generating a random DNA sequence, adding a single-ended adapter DNA sequence containing the known index at both ends of the random DNA sequence to obtain an internal reference sequence, and synthesizing a sequencing quality control sequence based on the internal reference sequence;
performing, based on the sequencing quality control sequence, high-throughput sequencing on a library of samples to be tested to obtain sequencing data; and
performing result analysis on the sequencing data to obtain a sample error distribution rate of the library of samples to be tested, and ending the high-throughput sequencing.

2. The method for high-throughput sequencing based on the internal reference with the known index according to claim 1, wherein synthesizing the sequencing quality control sequence based on the internal reference sequence comprises:
cloning the internal reference sequence into a pUC57 vector to obtain the sequencing quality control sequence.

3. The method for high-throughput sequencing based on the internal reference with the known index according to claim 2, wherein a nucleotide sequence of the sequencing quality control sequence is as shown in CS0002, wherein a nucleotide sequence of CS0002 is:

gaacgacatggctacgatccgacttcctgggtgagcatcaggggcgatacgttcacatcccggaggacctcgaagcacaacgccaggac

tgcgaagatccacgaaatcacgaaacagcacgacattgaaggttggactgatccagttgctgggtatgtatggatataagttcatcaagtcg

gaggccaagcggtcttaggaagacaataggtccgatcaactccttggctcaca.

4. The method for high-throughput sequencing based on the internal reference with the known index according to claim 2, wherein the nucleotide sequence of the sequencing quality control sequence is as shown in CS0003, wherein a nucleotide sequence of CS0003 is:

gaacgacatggctacgatccgacttagaatgaagtgggcacagttaagatctaataaaacgtggaaacagtcttgacgggctacgatcaga

gcgggacatcagtaactaatagccagtctcgtgcgaccgtaaccaagacgctcaccgtaataaccaggaggagtaacggcggggaagtc

ggaggccaagcggtcttaggaagacaaggacggaatccaactccttggctcaca.

5. The method for high-throughput sequencing based on the internal reference with the known index according to claim 2, wherein the nucleotide sequence of the sequencing quality control sequence is as shown in CS0004, wherein a nucleotide sequence of CS0004 is:

gaacgacatggctacgatccgacttttggtgttgttcattttacgaactgcacttgtacatttattacggcgaacgaattatcagtcccgaactgt
aatactgactgtaagttagttactaggagtaaattgcgtgagtataatggtaactacgtcatgctggactgtgttaccgaagtcggaggccaag
cggtcttaggaagacaacttactgccgcaactccttggctcaca.

6. The method for high-throughput sequencing based on the internal reference with the known index according to claim 2, wherein the nucleotide sequence of the sequencing quality control sequence is as shown in CS0005, wherein a nucleotide sequence of CS0005 is:

gaacgacatggctacgatccgacttgaggaaccagttcctcatacatacgtttacttaatgacagactcccttatacgtgttaacgttaatctctt
agtgttagtgagagtaaagttatatggtcattagataatagagggatgataaaatgtgaatcatcgagagacgatttctaagtcggaggccaa
gcggtcttaggaagacaaacctaattgacaactccttggctcaca.

7. The method for high-throughput sequencing based on the internal reference with the known index according to claim 2, wherein performing, based on the sequencing quality control sequence, the high-throughput sequencing on the library of samples to be tested to obtain the sequencing data comprises:
amplifying the sequencing quality control sequence by a designated PCR amplification primer to obtain a target sequencing quality control sequence; and
performing, based on the target sequencing quality control sequence, high-throughput sequencing on the library of samples to be tested to obtain the sequencing data.

8. The method for high-throughput sequencing based on the internal reference with the known index according to claim 7, wherein performing, based on the target sequencing quality control sequence, the high-throughput sequencing on the library of samples to be tested to obtain the sequencing data comprises:
determining an amount of adding the target sequencing quality control sequence to obtain an addition amount, and adding the target sequencing quality control sequence to the library of samples to be tested according to the addition amount to obtain a mixed library; and
performing a high-throughput sequencing operation on the mixed library to obtain the sequencing data.

9. The method for high-throughput sequencing based on the internal reference with the known index according to claim 1, wherein generating the random DNA sequence comprises:
generating the random DNA sequence whose size is equal to a size of a sample fragment in the library of samples to be tested minus a size of the two single-ended adapter DNA sequences.

10. The method for high-throughput sequencing based on the internal reference with the known index according to claim 1, wherein the random DNA sequence is a bacteriophage sequence or a phytogenic pathogen sequence, wherein both the bacteriophage sequence and the phytogenic pathogen sequence are gene sequences of non-pathogenic pathogens.

11. The method for high-throughput sequencing based on the internal reference with the known index according to claim 1, wherein generating the random DNA sequence comprises:
selecting a reverse virus sequence by a specific species screening algorithm; and
cutting the selected reverse virus sequence into a preset size, aligning a pathogen database and a host database, and determining the reverse virus sequence as the random DNA sequence in the case that the selected reverse virus sequence is not present in the pathogen database or the host database.

12. The method for high-throughput sequencing based on the internal reference with the known index according to claim 11, wherein cutting the selected reverse virus sequence into the preset size comprises:
cutting the selected reverse virus sequence into a size of 150 bp.

13. The method for high-throughput sequencing based on the internal reference with the known index according to claim 11, wherein aligning the pathogen database and the host database comprises:
aligning the pathogen database and the host database by BLASTN.

14. The method for high-throughput sequencing based on the internal reference with the known index according to claim 7, wherein amplifying the sequencing quality control sequence by the designated PCR amplification primer to obtain the target sequencing quality control sequence comprises:
a system for reaction for PCR amplification comprises: a sequencing quality control sequence, a buffer, dNTP, DNA polymerase, a mixed PCR extension primer, and ddH₂O.

15. The method for high-throughput sequencing based on the internal reference with the known index according to claim 14, wherein the sequencing quality control sequence has a volume of 2 µL, the buffer has a volume of 10 µL, the dNTP has a volume of 1 µL, the DNA polymerase has a volume of 0.5 µL, the mixed PCR extension primer has a volume of 5 µL, and the ddH₂O has a volume of 31.5 µL.

16. The method for high-throughput sequencing based on the internal reference with the known index according to claim 14, wherein the DNA polymerase is Q5 hot-start ultra-fidelity DNA polymerase.

17. The method for high-throughput sequencing based on the internal reference with the known index according to claim 14, wherein a reaction procedure for the PCR amplification comprises:
1) setting a temperature of a hot cover to 105°C;
2) carrying out the reaction by one cycle under a temperature of 95°C and a duration of 3 min;
3) carrying out the reaction under a temperature of 95°C and a duration of 20s;
4) carrying out the reaction under a temperature of 58°C and a duration of 20s;
5) carrying out the reaction under a temperature of 72°C and a duration of 20s;
6) carrying out the reaction by one cycle under a temperature of 72°C and a duration of 5 min;
7) holding the reaction under a temperature of 4°C;
wherein 30 cycles are performed in phases 3) to 5).

18. The method for high-throughput sequencing based on the internal reference with the known index according to claim 7, wherein upon amplifying the sequencing quality control sequence by the designated PCR amplification primer to obtain the target sequencing quality control sequence, the method further comprises:
detecting, by agarose gel electrophoresis, whether an amplification product is consistent with an expected product.

19. The method for high-throughput sequencing based on the internal reference with the known index according to claim 18, wherein detecting, by agarose gel electrophoresis, whether the amplification product is consistent with the expected product comprises:
detecting, by agarose electrophoresis and Qsep analysis, whether the target sequencing quality control sequence is consistent with an expected sequence, and performing, by the target sequencing quality control sequence, high-throughput sequencing on the library of samples to be tested in the case that the target sequencing quality control sequence is consistent with the expected sequence.

20. The method for high-throughput sequencing based on the internal reference with the known index according to claim 18, wherein detecting, by agarose gel electrophoresis, whether the amplification product is consistent with the expected product comprises:
formulating an agarose gel, adding a sample to the agarose gel, and carrying out an electrophoresis experiment by an electrophoresis apparatus to obtain an electrophoresis result image; and
determining, based on the electrophoresis result image, whether a band position is consistent with an expected position.
